# Europäisches Patentamt
## European Patent Office
## Office européen des brevets

(19)

(11) Veröffentlichungsnummer: **0 131 913**
**B1**

(12) **EUROPÄISCHE PATENTSCHRIFT**

(45) Veröffentlichungstag der Patentschrift:
24.09.86

(51) Int. Cl.⁴: **C 07 C 143/02,** C 07 C 139/14

(21) Anmeldenummer: **84108173.0**

(22) Anmeldetag: **12.07.84**

(54) **Verfahren zur schonenden Isolierung von Paraffinsulfonsäuren oder Paraffinsulfonaten aus den bei der Sulfoxidation von Paraffinen erhaltenen Reaktionsgemischen.**

(30) Priorität: **15.07.83 DE 3325517**

(43) Veröffentlichungstag der Anmeldung:
**23.01.85 Patentblatt 85/4**

(45) Bekanntmachung des Hinweises auf die Patenterteilung:
**24.09.86 Patentblatt 86/39**

(84) Benannte Vertragsstaaten:
**DE FR GB IT NL**

(56) Entgegenhaltungen:
**DE-A-1 568 591**
**DE-A-2 139 477**
**GB-A-347 164**

(73) Patentinhaber: **HOECHST AKTIENGESELLSCHAFT,**
**Postfach 80 03 20, D-6230 Frankfurt am Main 80**
**(DE)**

(72) Erfinder: **Pistorius, Rudolf, Dr., Neumühlstrasse 15,**
**D-6274 Hünstetten (DE)**

LIBER, STOCKHOLM 1986

## Beschreibung

Die durch Sulfoxidation von n-Paraffinen z.B. nach dem Verfahren des deutschen Patents 910 165 erhältlichen wäßrigen Lösungen von Paraffinsulfonsäuren enthalten außerdem noch Schwefeldioxid, Schwefelsäure und hydrotrop gelöste Paraffine. Um aus solchen Reaktionsgemischen brauchbare Paraffinsulfonsäuren bzw. Paraffinsulfonate von guter Qualität, d.h. möglichst schwefelsäure- bzw. salzarme, helle und weitestgehend geruchsfreie Produkte zu isolieren, müssen Schwefeldioxid, Schwefelsäure und Paraffine möglichst quantitativ und schonend abgetrennt werden. Die Paraffinsulfoxidationsprodukte beginnen sich bereits bei Temperaturen oberhalb 50°C zu zersetzen, was sich äußerlich an der Verfärbung des sauren Reaktionsgemisches von wasserhell über gelblich, braun bis schließlich tiefschwarz zeigt. Wenn auch die Menge der durch die Temperatureinwirkung zersetzten Paraffinsulfonsäure noch relativ gering ist, solange die sauren Reaktionsgemische nicht über längere Zeit Temperaturen über 100°C ausgesetzt sind, erfordert jedoch bereits ein kleiner Anteil an zersetzten Produkten wegen ihrer Farbintensität einen beträchtlichen Bleichaufwand, wenn man zu einwandfrei hellen Produkten kommen will.

Es wurde festgestellt, daß demgegenüber die schwach alkalisch reagierenden Salze der Paraffinsulfonsäuren relativ stabil sind. Temperaturen unter 200°C führen auch bei längerer Erhitzungsdauer nur zu ganz unwesentlichen Verfärbungen und auch höhere Temperaturen bis zu etwa 260°C ergeben Verfärbungen, die sich noch leicht mit geringen Bleichmittelmengen wieder beseitigen lassen.

Es muß daher bereits beim ersten Schritt der Aufarbeitung der Paraffin-Sulfoxidations-Reaktionsgemische, bei der Entgasung zur Entfernung des Schwefeldioxids, darauf geachtet werden, daß keine Verfärbung auftritt. Wird die Entgasung im schwachen Vakuum ausgeführt, so bedarf es nur eines kurzzeitigen Erwärmens auf etwa 85°C, um eine nahezu vollständige Eliminierung des Schwefeldioxids zu erreichen. Durch unmittelbar anschließendes Wiederabkühlen des Reaktionsgemisches auf Raumtemperatur kann bei diesem Prozeß eine merkliche Zersetzung, d.h. eine eintretende Farbvertiefung des Reaktionsgemisches verhindert werden.

In Einblick auf die Qualität des Paraffinsulfonats wäre es günstig, das Reaktionsgemisch nach der Entgasung sofort zu neutralisieren. Eine derartige Verfahrensweise ist jedoch wegen des zur Neutralisierung der Schwefelsäure erforderlichen hohen Verbrauchs an Alkali sowie wegen der beachtlichen Verluste an Paraffinsulfonat, die beim Abfiltrieren des Alkalisulfats auftreten, unwirtschaftlich und technisch nicht praktikabel.

Nach der Entfernung des Schwefeldioxids aus dem Reaktionsgemisch muß deshalb versucht werden, vor der Neutralisation die Schwefelsäure möglichst vollständig und unter Schonung der Paraffinsulfonate aus dem Gemisch abzutrennen. Bei den bekannten Verfahren, die ein solches Ziel anstreben, geht man in allgemeinen so vor, daß man das entgaste Sulfoxidationsgemisch mit einem geeigneten organischen Lösungsmittel behandelt, um eine Entmischung in eine organische Phase, welche die Paraffinsulfonsäuren enthält und in eine wäßrige Phase, welche die Schwefelsäure soweit wie möglich in Form einer im allgemeinen 10 bis 25%igen wäßrigen Lösung enthält, herbeizuführen. Die beiden Phasen werden dann getrennt und die organische Phase zur Isolierung der Paraffinsulfonsäuren bzw. ihrer Salze weiter aufgearbeitet. So ist es aus der am 29.1.1953 bekanntgemächten deutschen Patentanmeldung F 3718, 120 bereits bekannt, wasserunlösliche oder nur beschränkt mit Wasser mischbare organische Lösungsmittel, wie z.B. Bensol, Chlorbenzol, Cyclohexan, Tetrachlorkohlenstoff, Chloroform, Methylenchlorid und dergleichen zur Abtrennung von Schwefelsäure den Sulfoxidationsgemisch zuzusetzen. Gemäß DE-OS 27 30 245 kommen für den gleichen Zweck auch Ether wie z.B. Diethylether oder Di-n-butylether und gemäß DE-OS 27 45 691 auch Ketone oder Ester zur Anwendung.

Keines dieser bekannten Verfahren zur Abtrennung von Schwefelsäure bei niederen Temperaturen hat sich bislang technisch durchsetzen können, weil entweder der Anteil der abgeschiedenen Schwefelsäure zu gering oder aber der Aufwand zur destillativen Abtrennund des Lösungsmittels zu groß ist.

Außerdem ist es aus der DE-A-2 139 477 bekannt, die Schwefelsäure mit Alkoholen abzutrennen, die mindestens 5 C-Atome enthalten. Niedere Alkohole dagegen haben eine völlig andere Wirkung bei der Aufarbeitung derartiger Sulfoxidationsgemische, da sie nur die Abtrennung der nicht umgesetzten Paraffine bewirken. Eine Abtrennung der Schwefelsäure können diese niederen Alkohole für sich allein nicht bewirken.

Zur Herstellung von Paraffinsulfonsäuren nuß weiterhin das in Reaktionsdemisch vorhandene nicht umgesetzte Paraffin mit beträchtlichem Aufwand, z.B. durch Austreiben mit überhitztem Wasserdampf, entfernt verden. Als vorteilhafter hat sich eine Arbeitsweise herausgestellt, bei der zunächst mit einen Alkohol, z.B. Mehanol, Propanol oder Isopropanol der größte Teil des Paraffins abgeschieden wird, un dann eine Operation anzuschließen, die eine möglichst vollständige Abtrennung der Schwefelsäure von den Paraffinsulfonsäuren ermöglicht, ohne daß sich das Reaktionsgemisch durch Zersetzung der Sulfonierungsprodukte verfärbt und ohne daß sich Alkalisulfate bilden.

Es wurde nun gefunden, daß überraschend eine schonende Isolierung von Paraffinsulfonsäuren oder Paraffinsulfonaten aus den Paraffin-Sulfoxidations-Reaktionsgemischen und eine weitgehende Abscheidung der Schwefelsäure gelingt, wenn man das Reaktionsgemisch in an sich z.B. aus den deutschen Patentschriften 907 052, 910 165 und der deutschen Offenlegungsschrift 15 68 591 bekannten Weise vom überwiegenden Teil des Paraffins durch Zusatz von 2 oder 3 Kohlenstoffatome enthaltenden aliphatischen Alkoholen als obere Phase abtrennt und das Reaktionsgemisch danach zur Abscheidung der Schwefelsäure mit einem wasserunlöslichen oder nur beschränkt mit Wasser mischbaren schwach polaren organischen Lösungsmittel versetzt.

Gegenstand der Erfindung ist somit ein Verfahren zur schonenden Isolierung von Paraffinsulfonsäuren oder Paraffinsulfonaten aus den bei der Sulfoxidation von n-Paraffinen erhaltenen, durch Entgasung bei erhöhter Temperatur vom Schwefeldioxid befreiten Reaktionsgemischen, die höhermolekulare Paraffinsulfonsäuren, Schwefelsäure, n-Paraffine und Wasser enthalten, welches dadurch gekennzeichnet ist, daß man ein solches Reaktionsgemisch mit einem aliphatischen Alkohol mit 2 bis 3 Kohlenstoffatomen versetztund den als obere Phase abgeschiedenen überwiegenden Teil des Paraffins abtrennt und das Reaktionsgemisch danach mit einen weniger polaren wasserunlöslichen oder nur beschränkt mit Wasser mischbaren organischen Lösungsmittel vermischt, die entstandene, die Schwefelsäure enthaltende wäßrige Phase abtrennt und die so erhaltene Paraffinsulfonsäuren enthaltende Produktphase entweder zur Gewinnung der Paraffinsulfonsäuren eindampft und gegebenenfalls durch Zusatz von etwas Wasserstoffperoxid bleicht oder zur Gewinnung von Paraffinsulfonaten mit Alkali neutralisiert und gegebenenfalls unter Anwendung von überhitztem Wasserdampf zur Entfernung von Lösungsnitteln und gegebenenfalls restlichem Paraffin eindampft.

Bei der Isolierung von Paraffinsulfonsäuren gemäß dem Verfahren der vorliegenden Erfindung können im Falle nicht ausreichender Abscheidung der Paraffine durch den Zusatz der aliphatischen Alkohole die im Reaktionsgemisch noch vorhandenen restlichen Anteile der Paraffine mit niedrig siedenden Kohlenwasserstoffen wie z.B. Pentan, Hexan, Heptan usw. extrahiert werden. Nach dieser Extraktion erfolgt dann die Abscheidung der Schwefelsäure aus dem die aliphatischen Alkohole enthaltenden Reaktionsgemisch durch Zugabe des schwach polaren Lösungsmittels.

Als Ausgangsmaterial für das Verfahren der vorliegenden Erfindung können die durch schonende Entgasung von Schwefeldioxid befreiten Reaktionsgemische der bekannten Sulfoxidationsverfahren eingesetzt werden, die wäßrige Gemische von höhermolekularen Paraffinsulfonsäuren, Schwefelsäure und n-Paraffin darstellen. Beispielsweise können die nach dem Verfahren der deutschen Patentschrift 735 096 erhältlichen Reaktionsgemische verwendet werden. Es kann ferner auch von Reaktionsgemischen ausgegangen werden, wie sie bei der Sulfoxidation mit Hilfe von Peroxiden, Ozon oder auch mit ∂-Strahlen, z.B. nach dem Verfahren der deutschen Patentschrift 11 39 116 erhalten werden. Bevorzugt legt man für das vorliegende Verfahren die bei der Sulfoxidation von n-Paraffinen mit Kettenlängen von etwa 7 bis 20, vorzugsweise 13 bis 18 Kohlenstoffatomen anfallenden wäßrigen Reaktionsgemische zugrunde, die sich bei der Sulfoxidation als klare schwerere Phase von dem überschüssigen Paraffin abtrennen.

Die Abscheidung des Paraffins mit den 2 bis 3 Kohlenstoffatome enthaltenden aliphatischen Alkoholen Ethanol, n-Propanol oder Isopropanol, vorzugsweise Isopropanol, erfolgt zweckmäßigerweise bei Temperaturen in Bereich von 5 bis 50°C, vorteilhaft bei 10 bis 30°C. Die anzuwendende Menge an Alkoholen beträgt in Abhängigkeit von der Zusammensetzung des Reaktionsgemisches etwa 5 bis 80 Gew.-%, vorteilhaft etwa 8 bis 50 Gew.-%, insbesondere 10 bis 40 Gew.-%, bezogen auf das Gewicht des eingesetzten Reaktionsgemisches. Die Abscheidung des Paraffins als obere Phase erfolgt innerhalb von etwa 5 bis 90 Min, meist innerhalb von 8 bis 20 Min. Bei Verwendung von Isopropanol scheidet sich meist in einen engen Konzentrationsbereich außerdem etwas verdünnte Schwefelsäure als schwerste Phase ab, die als solche abgetrennt werden kann, jedoch nicht unbedingt abgetrennt werden muß.

In Falle der beabsichtigten Isolierung freier Paraffinsulfonsäuren ist es meist zweckmäßig, an dieser Stelle des Verfahrens aus dem die Alkohole enthaltenden Reaktionsgemisch die darin noch verbliebenen restlichen Paraffine mit niedrig-siedenden Kohlenwasserstoffen mit etwa 5 bis 8 C-Atomen zu extrahieren. Sofern für die Abscheidung der Paraffine ein $C_3$-Alkohol verwendet wurde, eignet sich als Kohlerwasserstoff für die nachfolgende Extraktion besonders gut n-Heptan, dessen Löslichkeit in der Propanol enthaltenden Produktphase besonders gering ist.

Für die nachfolgende Abscheidung der Schwefelsäure aus dem nunmehr aliphatischen Alkohol enthaltenden Reaktionsgemisch eignen sich wasserunlösliche oder nur beschränkt mit Wasser mischbare schwach polare organische Lösungsmittel, die unter den Bedingungen der Aufarbeitung gegenüber den Bestandteilen des Reaktionsgemisches inert sind. Es kommen organische Lösungsmittel in Betracht, deren Siedepunkt im Bereich von etwa 20 bis 150°C, vorzugsweise von 30 bis 100°C liegt. Geeignete Lösungsmittel dieser Art sind z.B. Tetrachlorkohlenstoff, Chloroform, 1,2-Dichlorethan, 1,1,2-Trichlor-trifluorethan und insbesondere Methylenchlorid. Die zuzusetzende Menge des schwach polaren organischen Lösungsmittels liegt im Bereich von etwa 10 bis 200 Gew.-%, vorteilhaft von 20 bis 150 Gew.-%, insbesondere bei 30 bis 70 Gew.-%, bezogen auf das Gewicht der eingesetzten Reaktionsmischung. Dabei sollte jedoch die Menge des schwach polaren organischen Lösungsmittels vorzugsweise gleich groß oder größer, insbesondere etwa doppelt so groß sein wie die Menge des verwendeten aliphatischen Alkohols.

Die Schwefelsäureabscheidung durch Zugabe der schwach polaren organischen Lösungsmittel erfolgt im allgemeinen bei Temperaturen im Bereich von etwa 5 bis 50°C, vorzugsweise bei etwa 10 bis 30°C. Nach Zumischen der organischen Lösungsmittel erfolgt die Phasentrennung innerhalb von längstens 2 Stunden; im allgemeinen jedoch bereits nach 15 bis 30 Min. Die Phasentrennungszeit kann durch geeignete Maßnahmen z.B. durch Passieren des Gemisches durch einen Tröpfchenabscheider entscheidend vorkürzt werden. Bereits die Filtration durch ein Papierfilter oder durch Glaswolle bewirkt eine wesentlich schnellere Trennung, so daß bereits nach etwa 10 Minuten die wäßrige, Schwefelsäure enthaltende Phase abgeschieden werden kann.

Sofern nach Abscheidung der Schwefelsäure eine noch weitergehende Entfernung restlicher Schwerelsäure aus der Produktphase gewünscht wird, so kann dies durch Zugabe von Kalk und etwa 10 bis 30 Minuten andauerndes Verrühren sowie Abfiltrieren des ausgefallenen Calciumsulfats erfolgen.

Zur Gewinnung der Paraffinsulfonsäuren wird die nach dem Abtrennen der wäßrigen Schwefelsäure nunmehr neben den Paraffinsulfonsäuren noch Paraffine, aliphatischen Alkohol und organisches Lösungsmittel enthaltende Produktphase mit Petroläther zur Entfernung der Paraffine extrahiert und anschließend soweit eingedampft, daß die Konzentration an Paraffinsulfonsäuren etwa 85 bis 95 Gew.-% beträgt. Durch Zusatz geringer Mengen Wasserstoffparoxid, z.B. von etwa 1 Gew.-% einer 50 %igen Wasserstoffperoxidlösung, erhält man bei Raumtemperatur nach einigen Stunden - bei erhöhter Temperatur von etwa 50°C nach einigen Minuten - sehr helle Paraffinsulfonsäuren.

Bei beabsichtigter Gewinnung von Paraffinsulfonaten wird die nach dem Abtrennen der wäßrigen Schwefelsäure neben Paraffinsulfonsäuren nunmehr noch aliphatischen Alkohol, organisches Lösungsmittel und gegebenenfalls restliche Paraffine enthaltende Produktphase bei Temperaturen unter 55°C, vorzugsweise unter 50°C vorteilhaft so neutralisiert, daß unter intensiven Rühren gleichzeitig Alkali und die Produktphase zusammengegeben werden. Dabei wird die Dosiergeschwindigkeit so eingestellt, daß der pH-Wert, gemessen mit einer geeichten pH-Glaselektrode, in Gemisch der beiden Flüssigkeiten immer oberhalb 7, insbesondere oberhalb 10,5 liegt, damit das schließlich am Ende der Aufarbeitung erhaltene Paraffinsulfonat einen neutralen bis schwach alkalischen pH-Wert aufweist. Es ist aber auch möglich, bei der Neutralisation die gesamte Alkalimenge vorzulegen und die Produktphase so lange zulaufen zu lassen, bis der gewünschte pH-Wert oberhalb 7 bzw. oberhalb 10,5 erreicht ist. Grundsätzlich möglich, wenn auch weniger vorteilhaft ist ein umgekehrtes Vorgehen, d.h. Zugabe des Alkalis zur vorgelegten Produktlösung.

Zur Neutralisation der Paraffinsulfonsäuren können beliebige Alkalien verwendet werden. Es können Natrium- oder Kaliumhydroxid zweckmäßig in Form konzentrierter wäßriger Lösungen genonmen werden; es ist jedoch auch möglich, Natrium- oder Kaliumcarbonat oder Natrium- oder Kaliumbicarbonat einzusetzen.

Nach der Neutralisation wird die Paraffinsulfonat enthaltende Produktphase zur Entfernung von Alkohol, organischem Lösungsmittel und gegebenenfalls restlichen Paraffinen in an sich bekannter Weise durch ein- oder mehrstufige Destillation eingedampft, wobei schließlich noch mit überhitzten Wasserdampf von 180 bis 280°C die restlichen Paraffine und gegebenenfalls Lösungsmittel ausgetrieben werden.

Die so gewonnene Paraffinsulfonat-Schmelze kann anschließend durch Zugabe von Wasser bei 100 bis 160° unter entsprechenden Druck auf die gewünschte Konzentration eingestellt werden, wobei man dem Wasser zur Beseitigung eventueller Farb- und Geruchsspuren noch 0,01 bis 1 Gew.-%, insbesondere 0,1 bis 0,3 Gew.-% Wasserstoffperoxid zumischen kann.

Ein wesentlicher Vorteil der erfindungsgemäßen Arbeitsweise ist darin zu sehen, daß es hiermit gelingt, helle, geruchsund schwefelsäure- bzw. salzarme Produkte auf wirtschaftliche Weise zu erhalten, was vor allem durch die weitgehende Abscheidung der Schwefelsäure unter außerordentlich milden Bedingungen bewirkt wird. Die bei dem Verfahren der Erfindung erreichte weitgehende Abscheidung der Schwefelsäure durch Einwirkung von wenig polaren organischen Lösungsmitteln in Gegenwart von aliphatischen Alkoholen ist deshalb überraschend, weil jedes dieser Lösungsmittel für sich allein nur eine weit unvollständigere oder auch überhaupt keine Abscheidung von Schwefelsäure bewirkt, so daß dieser Kombinationseffekt nicht zu erwarten war. Wegen der gegenüber Alkoholen wesentlich kleineren Verdampfungsenergie der schwach polaren organischen Lösungsmittel erweist es sich weiterhin als vorteilhaft, daß bereits Alkoholmengen unter 40 Gew.-%, in geeigneten Fällen auch unter 20 Gew.-%, bezogen auf die Menge des Reaktionsgemisches, ausreichen, um Paraffinsulfonate mit einen Gehalt an anorganischen Salzen unter 5 % (bezogen auf die waschaktive Substanz) bzw. Paraffinsulfonsäuren mit entsprechend niedrigem Schwefelsäuregehalt zu erhalten.

## Beispiel 1

In einer Sulfoxidationsapparatur wurden geradkettige Paraffinkohlenwasserstoffe mit 14 bis 17 Kohlenstoffatomen mit Schwefeldioxid und Sauerstoff in Gegenwart von Wasser unter Bestrahlung mit UV-Licht bei 30 bis 40°C umgesetzt. Es wurde ein wäßriges Reaktionsgemisch erhalten, das durch Erwärmen auf 85°C unter schwachem Vakuum vom Schwefeldioxid befreit wurde. Hiernach bestand das Reaktionsgemisch aus 40,3 Gew.-% Wasser, 7,57 Gew.-% Schwefelsäure, 22,12 Gew.-% Paraffinsulfonsäuren und 30,01 Gew.-% Paraffinen.

3000 g dieses entgasten Reaktionsgemisches wurden abgekühlt und mit 450 g Isopropanol versetzt. Die erhaltene Mischung hatte eine Temperatur von 25°C. Innerhalb von 15 Minuten trennte sich das Gemisch in drei Phasen auf. Es schieden sich 662 g einer oberen Phase ab, die zu 99 Gew.-% aus Paraffinen und zu 1 Gew.-% aus Isopropanol bestand. Als untere Schicht trennten sich 268 g ab, die 17,9 Gew-.-% Schwefelsäure und 8,95 Gew.-% Isopropanol enthielten; der Gehalt an Alkansulfonsäuren lag deutlich unter 0,1 Gew.-%. In die 2520 g umfassende mittlere Phase wurden 1350 g Methylenchlorid eingerührt und das Gemisch in einen Abscheider filtriert. Innerhalb von 20 Minuten schieden sich 848 g einer 17,8 gew.-%igen Schwefelsäure ab. Diese Phase enthielt ebenfalls noch 8,95 Gew.-% Isopropanol und 0,6 Gew.-% Methylenchlorid mit 0,1 Gew.-% Paraffinsulfonsäuren. Die verbliebene, 3022 g umfassende Produktphase wurde mit 222 g·50%iger Natronlauge so neutralisiert, daß die Natronlauge und die Isopropanol und Methylenchlorid enthaltende Produktphase gleichzeitig unter starkem Rühren in ein Reaktionsgefäß liefen, wobei die Dosiergeschwindigkeit der Natronlauge so eingestellt wurde, daß der pH-Wert im Reaktionsgefäß immer oberhalb 12 lag. Nach Beendigung der Neutralisation wurde der pH-Wert durch weitere Natronlaugezugabe auf 13,5 eingestellt.

Die so erhaltene, Paraffinsulfonat enthaltende, nur schwach gelblich gefärbte Produktphase, wurde zunächst bis zu einer Sumpftemperatur von 96°C eingedampft. Das Konzentrat (1600 g) wurde anschließend kontinuierlich in einem auf 160 bis 170°C Sumpftemperatur erhitzten Kolben einer Destillationsanlage eindosiert, wobei 500 g Wasser, 100 g Isopropanol und 46 g Paraffine mit übergingen. Durch Nachstrippen mit 1000 g auf 200°C überhitztem Wasserdampf konnten aus der auf 190°C gehaltenen Schmelze noch 195 g Paraffine ausgetrieben werden.

Durch Zugabe von 438 g Wasser und 3 g einer 50%igen Wasserstoffperoxidlösung bei 100°C wurden 1200 g einer 60%igen Paraffinsulfonatpaste erhalten, die noch 2,4 Gew.-% Natriumsulfat und 0,3 Gew.-% Paraffine enthielt und die nahezu farb- und geruchlos war.

## Beispiel 2

Es wurde in gleicher Weise wie in Beispiel 1 gearbeitet, die Menge des zugesetzten Methylenchlorids jedoch von 1350 g auf 1950 g heraufgesetzt. Die auf diese Weise erhaltene 60%ige Paraffinsulfonatpaste enthielt nur noch 2,1 Gew.-% Natriumsulfat.

## Beispiel 3

Es wurde von einen entgasten Sulfoxidationsreaktionsgemisch der folgenden Zusammensetzung ausgegangen: 40,5 Gew.-% Wasser, 7,2 Gew.-% Schwefelsäure, 21,7 Gew.-% Paraffinsulfonsäuren und 30,6 Gew.-% Paraffine.

4000 g dieses Gemisches wurden bei 20°C mit 600 g Isopropanol versetzt. Als obere Phase schieden sich 848 g Paraffin ab. Die beiden unteren Phasen wurden zusammen mit 1200 g Methylenchlorid vermischt, wonach sich insgesamt 1496 g wäßrige Schwefelsäure abtrennten. Die obere, die Paraffinsulfonsäuren enthaltende Phase wurde nun in zwei gleiche Anteile aufgeteilt, wovon die eine Hälfte wie im Beispiel 1 beschrieben, weiter aufgearbeitet wurde. Hierbei wurde eine 60%ige Paraffinsulfonat-Paste erhalten, die noch 3 Gew.-% Natriumsulfat und 0,8 Gew.-% Paraffine enthielt.

Die andere Hälfte der Produktphase wurde mit 10,1 g Kalk (Kalziumhydroxid) 15 Min. lang gerührt und der ausgefallene Gips abfiltriert. Das Filtrat wurde mit 128 g Natronlauge neutralisiert, wobei sich die Lösung infolge noch ausgefallenen Kalziumsulfates wieder leicht trübte. Es wurde nochmals filtriert und die weitere Aufarbeitung wie in Beispiel 1 vorgenommen. Es wurde eine 60%ige Paraffinsulfonat-Paste erhalten, die noch 0,9 Gew.-% Natriumsulfat und 0,8 Gew.-% Paraffine enthielt.

## Beispiel 4

1 kg eines entgasten Paraffin-Sulfoxidations-Reaktionsgemisches der Zusammensetzung 39,1 Gew.-% Wasser, 23,63 Gew.-% Paraffinsulfonsäuren, 7,57 Gew.-% Schwefelsäure und 29,7 Gew.-% Paraffine wurde mit 350 g Isopropanol versetzt. Nach 15 Minuten wurde die obere Phase, enthaltend 270 g Paraffine und 3 g Isopropanol, abgetrennt. Die untere Produktphase wurde mit 600 g n-Heptan versetzt und auf 75°C erwärmt, wodurch sich 344 g Schwefelsäure abschieden, die abgetrennt wurden. Danach wurde die Produktphase zur Entfernung des restlichen Paraffins viermal mit je 200 g n-Heptan bei Raumtemperatur extrahiert. Die Produktphase wurde nunmehr mit 400 g Methylenchlorid gemischt; die hierdurch abgeschiedenen 71,4 g wäßrige Schwefelsäure wurden abgetrennt. Anschließend wurde die Produktphase bis zu einem Paraffinsulfonsäuregehalt von 85 Gew.-% eingedampft. Der Gehalt dieses Produktes an Schwefelsäure betrug noch 1,9 Gew.-%. Durch Zusatz von 1 Gew.-% einer 50%igen Wasserstoffperoxidlösung wurde eine helle Paraffinsulfonsäure erhalten.

**Patentansprüche**

1. Verfahren zur schonenden Isolierung von Paraffinsulfonsäuren oder Paraffinsulfonaten aus wäßrigen, bei der Sulfoxidation von n-Paraffinen anfallenden Reaktionsgemischen, die durch Entgasung bei erhöhter Temperatur vom Schwefeldioxid befreit wurden und die höhermolekulare Sulfonsäuren, Schwefelsäure, n-Paraffine und Wasser enthalten, dadurch gekennzeichnet, daß man das Reaktionsgemisch mit einem aliphatischen Alkohol mit 2-3 Kohlenstoffatomen versetzt und die als obere Phase abgeschiedenen Paraffine abtrennt und das Reaktionsgemisch danach mit einen weniger polaren wasserunlöslichen oder nur beschränkt mit Wasser mischbaren organischen Lösungsmittel vermischt, die abgeschiedene, wäßrige Schwefelsäure enthaltende Phase abtrennt und die so erhaltene Paraffinsulfonsäuren enthaltende Produktphase entweder zur Gewinnung der Paraffinsulfonsäuren eindampft und gegebenenfalls durch Zusatz von etwas Wasserstoffperoxid bleicht oder zur Gewinnung von Paraffinsulfonaten mit Alkali neutralisiert und gegebenenfalls unter Anwendung von überhitztem Wasserdampf zur Entfernung von Lösungsmitteln und gegebenenfalls restlichem Paraffin eindampft.

2. Verfahren nach Anspruch 1, dadurch gekennzeichnet, daß man bei der Isolierung von Paraffinsulfonsäuren im Falle nicht ausreichender Abscheidung der Paraffine durch den Zusatz der aliphatischen Alkohole die im Reaktionsgemisch noch vorhandenen restlichen Anteile an Paraffinen mit niedrig siedenden Kohlenwasserstoffen mit 5 bis 8 Kohlenstoffatomen extrahiert und erst danach die Abscheidung der Schwefelsäure aus dem Reaktionsgemisch durch Zugabe des schwach polaren Lösungsmittels vornimmt.

3. Verfahren nach Anspruch 1, dadurch gekennzeichnet, daß die Einwirkung der Alkohole und der organischen Lösungsmittel zur Abscheidung von Paraffinen und wäßriger Schwefelsäure bei Temperaturen unter 50° C erfolgt.

4. Verfahren nach Anspruch 1, dadurch gekennzeichnet, daß die aliphatischen Alkohle und organischen Lösungsmittel im Gewichtsverhältnis 1:1 bis 1:2 angewandt werden.

5. Verfahren nach Anspruch 1, dadurch gekemnzeichnet, daß als aliphatischer Alkohol Isopropanol zugegeben wird.

6. Verfahren nach Anspruch 1, dadurch gekennzeichnet, daß als wasserunlösliches oder nur beschränkt mit Wasser mischbares organisches Lösungsmittel Methylenchlorid zugegeben wird.

**Claims**

1. A process for the isolation under mild conditions of paraffinsulfonic acids or paraffinsulfonates from aqueous reaction mixtures which are obtained from the sulfoxidation of n-paraffins, from which sulfur dioxide has been removed by degassing at elevated temperature, and which contain higher molecular weight sulfonic acids, sulfuric acid, n-paraffins and water, which process comprises adding an aliphatic alcohol having 2-3 carbon atoms to the reaction mixture, removing the paraffins which separate out as the upper phase, and then mixing the reaction mixture with a less polar organic solvent which is insoluble in water or is miscible with water to only a limited extent, removing the aqueous phase which has separated out and contains sulfuric acid, and either evaporating the product phase containing paraffinsulfonic acids thus obtained to obtain the paraffinsulfonic acids and, where appropriate, bleaching by addition of a little hydrogen peroxide, or neutralizing with alkali to obtain paraffinsulfonates and evaporating with, where appropriate, the use of superheated steam to remove solvents and, where appropriate, remaining paraffin.

2. The process as claimed in claim 1, wherein during the isolation of paraffinsulfonic acids, in the case where the paraffins are not adequately separated out by the addition of the aliphatic alcohols, the remaining portions of paraffins still present in the reaction mixture are extracted with low boiling hydrocarbons having 5 to 8 carbon atoms, and only then is the separation out of the sulfuric acid from the reaction mixture by addition of the weakly polar solvent carried out.

3. The process as claimed in claim 1, wherein the action of the alcohols and the organic solvents to separate out paraffins and aqueous sulfuric acid takes place at temperatures below 50° C.

4. The process as claimed in claim 1, wherein the aliphatic alcohols and organic solvents are used in the weight ratio 1:1 to 1:2.

5. The process as claimed in claim 1, wherein isopropanol is added as the aliphatic alcohol.

6. The process as claimed in claim 1, wherein methylene chloride is added as the organic solvent which is insoluble in water or is miscible with water to only a limited extent.

## Revendications

1. Procédé pour isoler de façon ménagée les acides paraffine sulfoniques ou des paraffine sulfonates de mélanges réactionnels aqueux obtenus lors de la sulfoxydation de n-paraffines, dont on élimine, par dégazage à température élevée, l'anhydride sulfureux et qui contiennent les acides sulfoniques à poids moléculaire élevé, l'acide sulfurique, les n-paraffines et de l'eau, procédé caractérisé en ce qu'on ajoute au mélange réactionnel un alcool aliphatique comportant 2 à 3 atomes de carbone et l'on enléve les paraffines, sous forme de la phase supérieure séparée, puis l'on incorpore au mélange réactionnel un solvant organique peu polaire, insoluble dans l'eau ou qui n'est miscible à l'eau que de façon limitée, on enlève la phase aqueuse qui s'est séparée et contient de l'acide sulfurique et l'on concentre par évaporation la phase das produits, contenant les acides paraffine sulfoniques, que l'on obtient ainsi, pour obtenir les acides paraffine sulfonique et on les blanchit éventuellement par addition d'un peu de peroxyde d'hydrogène ou bien, pour obtenir des paraffine sulfonates, on neutralise avec une substance alcaline éventuellement en utilisant de la vapeur d'eau surchauffée, on élimine les solvants et en concentrant par évaporation éventuellement la paraffine restante.

2. Procédé selon la revendication 1, caractérisé en ce que, lors de l'isolement das acides paraffine sulfoniques et en cas de séparation insuffisante des paraffines par l'addition des alcools aliphatiques, on extrait à l'aide d'hydrocarbures à bas point d'ébullition, comportant 5 à 8 atomes de carbone, les fractions restantes de paraffines présentes encore dans le mélange réactionnel et ce n'est qu'ensuite que l'on effectue la séparation de l'acide sulfurique du mélange réactionnel, par addition du solvant faiblement polaire.

3. Procédé selon la revendication 1, caractérisé en ce qu'on fait agir les alcools et le solvant organique, pour séparer les paraffines et l'acide sulfurique aqueux, à des températures inférieures à 50° C.

4. Procédé selon la revendication 1, caractérisé en ce qu'on applique les alcools aliphatiques et les solvants organiques selon un rapport pondéral allant de 1:1 à 1:2.

5. Procédé selon la revendication 1, caractérisé en ce qu'on ajoute de l'isopropanol comme alcool aliphatique.

6. Procédé selon la revendication 1, caractérisé en ce qu'on ajoute du chlorure de méthylène comme solvant organique insoluble dans l'eau ou ne présentant qu'une miscibilité limitée avec l'eau.